# EUROPÄISCHE PATENTSCHRIFT

(11) **EP 0 753 501 B1**
(45) Veröffentlichungstag und Bekanntmachung des Hinweises auf die Patenterteilung: **04.11.1998**
(21) Anmeldenummer: 96107035.6
(22) Anmeldetag: 04.05.1996
(51) Int. Cl.: C07C 45/62, C07C 47/02

(54) **Verfahren zur Herstellung von 2-Ethylhexanal**
Process for the preparation of 2-ethylhexanal
Procédé de préparation du 2-éthylhexanal

(30) Priorität: 08.07.1995 DE 19524970
(43) Veröffentlichungstag der Anmeldung: 15.01.1997
(73) Patentinhaber: HÜLS AKTIENGESELLSCHAFT, 45764 Marl (DE)
(72) Erfinder: Büschken, Wilfried, Dr., 45721 Haltern (DE); Hummel, Jürgen, 45768 Marl (DE)

(56) Entgegenhaltungen:
- EP-A- 0 011 906
- US-A- 4 394 525

## Beschreibung

Die vorliegende Erfindung betrifft ein Verfahren zur Herstellung von 2-Ethylhexanal durch katalytische Hydrierung von 2-Ethylhex-2-enal.

2-Ethylhexanal wird für die Synthese von Riechstoffen verwendet und ist Vorstufe für 2-Ethylhexansäure.

Verfahren zur kontinuierlichen Herstellung von 2-Ethylhexanal durch eine katalytische Hydrierung von 2-Ethylhexenal sind bekannt:

Um bei einer kontinuierlichen Hydrierung einen nahezu vollständigen Umsatz zu erreichen, gibt es im Prinzip zwei Verfahren:
a) Es wird im Rohrreaktor (Reaktionsrohr mit ausreichender Länge) im geraden Durchgang hydriert.
b) Die Hydrierung wird in zwei Reaktoren durchgeführt. Dem ersten Reaktor, der meistens in Schlaufenfahrweise betrieben wird, ist ein zweiter Reaktor, in dem im geraden Durchgang hydriert wird, zum Finishen nachgeschaltet.

In der DE-OS 19 41 634 wird die Hydrierung an einem speziell hergestellten Pd/SiO₂-Katalysator beansprucht. Die Reaktion wird einstufig in Schlaufenfahrweise durchgeführt. Die Durchsätze liegen zwischen 0,4 - 0,6 [kg/l Katalysator · h]. Laut Beispiel beträgt bei einem Umsatz von 99,1 % die Selektivität für 2-Ethylhexanal 97,8 %.

In der DE-OS 20 08 128 wird ein einstufiges Schlaufenverfahren beschrieben, bei dem als besonderer Vorteil die Anwendung einer Oberflächenbelastung des Katalysators von 10 - 50 m/h herausgestellt wird. Bei einem Durchsatz von 0,325 [l/kg Katalysator · h] wird ein Umsatz von 99,2 % erreicht. Eine Selektivitätsangabe ist nicht zu entnehmen.

In den beiden vorangehenden Schriften ist die Raum-/Zeit-Ausbeute unter wirtschaftlichen Gesichtspunkten zu gering.

In der Patentschrift US 4 018 831 wird die Flüssigphasehydrierung an einem Nickel-Katalysator beschrieben. Bei einem Durchsatz von 1,1 [kg/kg Katalysator · h] wird ein nahezu 100 %iger Umsatz und eine Selektivität von 97,5 % erreicht. Hier ist die Selektivität nicht zufriedenstellend.

Der vorliegenden Erfindung lag daher die Aufgabe zugrunde, ein Verfahren bereitzustellen, das durch katalytische Hydrierung von 2-Ethylhex-2-enal ermöglicht, 2-Ethylhexanal in wirtschaftlicher Weise und mit hoher Selektivität herzustellen.

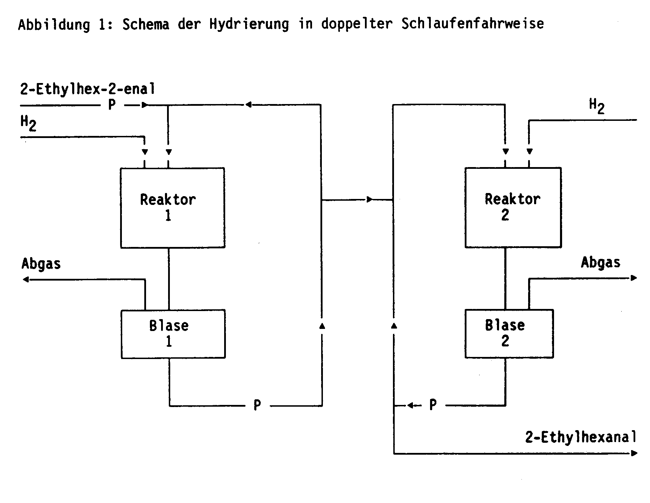

Es wurde nun überraschend gefunden, daß ein nahezu vollständiger Umsatz und eine hervorragende Selektivität erreicht wird, wenn man die katalytische Hydrierung in mehreren hintereinander geschalteten Schlaufen, bevorzugt in doppelter Schlaufenfahrweise (vgl. Abbildung 1), durchführt. Geeigneterweise wird dabei 2-Ethylhex-2-enal in einen Teil des Hydrieraustrags des ersten Reaktors eingespeist und dieses Gemisch auf den Kopf des ersten Reaktors geleitet. Aus der Hydriervorlage des ersten Reaktors wird standgeregelt Hydrierprodukt in die Schlaufe des zweiten Reaktors gefahren. Dieses Gemisch fließt auf den Kopf des zweiten Reaktors. Aus der Hydriervorlage des zweiten Reaktors wird standgeregelt Hydriergut abgefahren.

Das vorliegende Verfahren, beispielweise mit doppelter Schlaufenfahrweise, hat im Vergleich zur herkömmlichen Verfahrensweise (1. Stufe Schlaufe; 2. Stufe gerader Durchgang) den Vorteil, daß bei gleicher Katalysatormenge (Summe der Mengen in beiden Reaktoren) die Produktionsmenge um mehr als 30 % erhöht werden kann. Bei Umsätzen von über 99,9 % liegen die erreichten Selektivitäten für 2-Ethylhexanal bei über 99 %.

Gegenstand der vorliegenden Erfindung ist daher ein Verfahren zur Herstellung von 2-Ethylhexanal durch katalytische Hydrierung von 2-Ethylhex-2-enal, das dadurch gekennzeichnet ist, daß die Hydrierung in mindestens zwei hintereinander geschalteten Schlaufen durchgeführt wird, wobei 2-Ethylhex-2-enal zusammen mit einem Teil des Hydrierproduktes des 1. Reaktors auf den Kopf des 1. Reaktors geleitet, der restliche Teil des Hydrierproduktes des 1. Reaktors zusammen mit einem Teil des Hydrierproduktes des 2. Reaktors auf den Kopf des 2. Reaktors geleitet und, sofern mehr als zwei Schlaufen vorgesehen werden, der restliche Teil des Hydrierproduktes des 2. Reaktors zusammen mit einem Teil des Hydrierproduktes des folgenden Reaktors auf den Kopf des folgenden Reaktors geleitet wird usw. und das 2-Ethylhexanal aus dem restlichen Teil des Hydrierproduktes des letzten Reaktors gewonnen wird.

Bevorzugt wird beim erfindungsgemäßen Verfahren die Hydrierung in zwei hintereinander geschalteten Schlaufen durchgeführt, wobei 2-Ethylhex-2-enal zusammen mit einem Teil des Hydrierproduktes des 1. Reaktors auf den Kopf des 1. Reaktors geleitet, der restliche Teil des Hydrierproduktes des 1. Reaktors zusammen mit einem Teil des Hydrierproduktes des 2. Reaktors auf den Kopf des zweiten Reaktors geleitet und das 2-Ethylhexanal aus dem restlichen Teil des Hydrierproduktes des 2. Reaktors gewonnen wird.

Die Hydrierung kann beim erfindungsgemäßen Verfahren in allen Reaktoren sowohl in turbulenter als auch in laminarer Strömung durchgeführt werden. Es kann auch vorteilhaft sein, die Hydrierung zumindest im ersten Reaktor in turbulenter Strömung durchzuführen. Darüber hinaus wird hier in allen Reaktoren bevorzugt in flüssiger Phase hydriert.

Vorzugsweise führt man die Hydrierung beim erfindungsgemäßen Verfahren bei Temperaturen von 40 bis 150 °C durch, besonders vorzugsweise bei Temperaturen von 80 bis 130 °C. Ferner kann es von Vorteil sein, die Hydrierung in den Reaktoren auf unterschiedlichen Temperaturniveaus durchzuführen, beispielsweise indem man den zweiten bzw. folgenden Reaktor bei einer niedrigeren Temperatur betreibt als den ersten bzw. vorangehenden Reaktor.

Im allgemeinen arbeitet man beim erfindungsgemäßen Verfahren unter Druck. Bevorzugt wird hier die Hydrierung in den Reaktoren bei Drücken von 1 bis 100 bar abs. durchgeführt, besonders vorzugsweise bei Drücken von 5 bis 20 bar abs.. Darüber hinaus kann es von Vorteil sein, die Hydrierung in den Reaktoren des erfindungsgemäßen Verfahrens auf unterschiedlichen Niveaus durchzuführen, beispielsweise indem man den zweiten bzw. folgenden Reaktor bei einem niedrigeren Druck betreibt als den ersten bzw. vorangehenden Reaktor.

Als Katalysatorbestückung in den Reaktoren können gängige, hierfür geeignete Hydrierkatalysatoren eingesetzt werden, beispielsweise 0,5 % Pd/Al₂O₃ (Fa. Engelhard).

Bevorzugt wird beim erfindungsgemäßen Verfahren die Hydrierung in den Reaktoren an einem Palladium-Katalysator durchgeführt; besonders vorzugsweise ist dabei der Palladium-Katalysator auf einen Träger aus Aluminiumoxid aufgebracht.

Das erfindungsgemäße Verfahren ermöglicht eine besonders wirtschaftliche Herstellung von 2-Ethylhexanal mit sehr guter Ausbeute und hervorragend hoher Selektivität.

Das folgende Beispiel soll die vorliegende Erfindung näher erläutern, ohne ihren Anwendungsumfang einzuengen:

### Beispiel 1

Die Hydrierapparatur (vgl. Abbildung 1) hat zwei baugleiche Reaktoren mit folgenden Abmessungen: 0̸_{c} = 42 mm; l = 1 500 mm. Beide Reaktoren sind jeweils mit 1,9 l Katalysator (0,5 % Pd/Al₂O₃) gefüllt.

Technisches 2-Ethylhexenal (96 % 2-Ethylhexenal, 0,02 % 2-Ethylhexanol) wird verdünnt mit teilhydriertem Einsatzstoff (aus Blase 1) auf den Kopf des ersten Reaktors gefahren (1. Schlaufe). Aus Blase 1 wird Hydriergut standgeregelt in die Schlaufen des zweiten Reaktors gefahren. Aus der Blase 2 wird standgeregelt technisches 2-Ethylhexanal abgefahren.

In den Reaktoren liegen folgende Reaktionsbedingungen vor:

| | Reaktor 1 | Reaktor 2 |
|---|---|---|
| Durchsatz | 6,19 kg/h | 6,26 kg/h |
| Umlauf | 130 kg/h | 65 kg/h |
| H₂-Druck | 15 bar | 15 bar |
| mittlere Temperatur | 124 °C | 116 °C |
| Abgasmenge | 220 Nl/h | 110 Nl/h |

Nach Einstellung des quasistationären Zustandes wird ein Hydriergut folgender Qualität erhalten:

| | |
|---|---|
| 2-Ethylhexenal | 0,09 Gew.-% |
| 2-Ethylhexanal | 95,46 Gew.-% |
| 2-Ethylhexanol | 0,46 Gew.-% |

Dies entspricht einem Umsatz von 99,9 % und einer Selektivität von 99,4 %.

### Vergleichsbeispiel:

Im Gegensatz zum Beispiel 1 ist der Umlauf des zweiten Reaktors außer Betrieb (Umlaufmenge 0). Unter Einhaltung der Randbedingungen (Umsatz ≥ 99,9 % und Selektivität > 99 %) und Anstrebung des größtmöglichen Durchsatzes wurden folgende Reaktionsbedingungen gefunden:

| | Reaktor 1 | Reaktor 2 |
|---|---|---|
| Durchsatz | 4,74 kg/h | ≤ 4,79 kg/h |
| Umlauf | 130 kg/h | 0 kg/h |
| H₂-Druck | 15 bar | 15 bar |
| mittlere Temperatur | 100 °C | 90 °C |
| Abgasmenge | 160 Nl/h | 60 Nl/h |

Daraus geht hervor, daß bei Anwendung der doppelten Schlaufenfahrweise in der vorliegenden Apparatur 30 % mehr 2-Ethylhexanal erzeugt werden kann.

## Patentansprüche

1. Verfahren zur Herstellung von 2-Ethylhexanal durch katalytische Hydrierung von 2-Ethylhex-2-enal,
dadurch gekennzeichnet,
daß die Hydrierung in mindestens zwei hintereinander geschalteten Schlaufen durchgeführt wird, wobei 2-Ethylhex-2-enal zusammen mit einem Teil des Hydrierproduktes des 1. Reaktors auf den Kopf des 1. Reaktors geleitet, der restliche Teil des Hydrierproduktes des 1. Reaktors zusammen mit einem Teil des Hydrierproduktes des 2. Reaktors auf den Kopf des 2. Reaktors geleitet und, sofern mehr als zwei Schlaufen vorgesehen werden, der restliche Teil des Hydrierproduktes des 2. Reaktors zusammen mit einem Teil des Hydrierproduktes des folgenden Reaktors auf den Kopf des folgenden Reaktors geleitet wird usw. und das 2-Ethylhexanal aus dem restlichen Teil des Hydrierproduktes des letzten Reaktors gewonnen wird.

2. Verfahren nach Anspruch 1,
dadurch gekennzeichnet,
daß die Hydrierung in zwei hintereinander geschalteten Schlaufen durchgeführt wird, wobei 2-Ethylhex-2-enal zusammen mit einem Teil des Hydrierproduktes des 1. Reaktors auf den Kopf des 1. Reaktors geleitet, der restliche Teil des Hydrierproduktes des 1. Reaktors zusammen mit einem Teil des Hydrierproduktes des 2. Reaktors auf den Kopf des zweiten Reaktors geleitet und das 2-Ethylhexanal aus dem restlichen Teil des Hydrierproduktes des 2. Reaktors gewonnen wird.

3. Verfahren nach den Ansprüchen 1 und 2,
dadurch gekennzeichnet,
daß in allen Reaktoren in flüssiger Phase hydriert wird.

4. Verfahren nach den Ansprüchen 1 bis 3,
dadurch gekennzeichnet,
daß die Strömung in allen Reaktoren laminar ist.

5. Verfahren nach den Ansprüchen 1 bis 3,
dadurch gekennzeichnet,
daß die Strömung in allen Reaktoren turbulent ist.

6. Verfahren nach den Ansprüchen 1 bis 5,
dadurch gekennzeichnet,
daß die Hydrierung bei Temperaturen von 40 bis 150 °C durchgeführt wird.

7. Verfahren nach Anspruch 6,
dadurch gekennzeichnet,
daß die Hydrierung bei Temperaturen von 80 bis 130 °C durchgeführt wird.

8. Verfahren nach den Ansprüchen 1 bis 7,
dadurch gekennzeichnet,
daß die Hydrierung in den einzelnen Reaktoren auf unterschiedlichen Temperaturniveaus durchgeführt wird.

9. Verfahren nach den Ansprüchen 1 bis 8,
dadurch gekennzeichnet,
daß die Hydrierung bei Drücken von 1 bis 100 bar abs. durchgeführt wird.

10. Verfahren nach Anspruch 9,
dadurch gekennzeichnet,
daß die Hydrierung bei Drücken von 5 bis 20 bar abs. durchgeführt wird.

11. Verfahren nach den Ansprüchen 1 bis 10,
dadurch gekennzeichnet,
daß die Hydrierung in den einzelnen Reaktoren auf unterschiedlichen Druckniveaus durchgeführt wird.

12. Verfahren nach den Ansprüchen 1 bis 11,
dadurch gekennzeichnet,
daß die Hydrierung an einem Palladium-Katalysator durchgeführt wird.

13. Verfahren nach Anspruch 12,
dadurch gekennzeichnet,
daß die Hydrierung an einem Palladium-Katalysator durchgeführt wird, der auf einen Träger aus Aluminiumoxid aufgebracht ist.

## Claims

1. A process for the preparation of 2-ethylhexanal by catalytic hydrogenation of 2-ethylhex-2-enal, characterized in that the hydrogenation is carried out in at least two series-connected loops, 2-ethylhex-2-enal, together with a portion of the hydrogenation product from the first reactor, being passed to the top of the first reactor, the remainder of the hydrogenation product from the first reactor, together with a portion of the hydrogenation product from the second reactor, being passed to the top of the second reactor, and, if more than two loops are provided, the remainder of the hydrogenation product from the second reactor, together with a portion of the hydrogenation product from the following reactor, being passed to the top of the following reactor, etc., and 2-ethylhexanal being obtained from the remainder of the hydrogenation product from the last reactor.

2. A process according to claim 1, characterized in that the hydrogenation is carried out in two series-connected loops, 2-ethylhex-2-enal, together with a portion of the hydrogenation product from the first reactor, being passed to the top of the first reactor, the remainder of the hydrogenation product from the first reactor, together with a portion of the hydrogenation product from the second reactor, being passed to the top of the second reactor, and 2-ethylhexanal being obtained from the remainder of the hydrogenation product from the second reactor.

3. A process according to either of claims 1 and 2, characterized in that hydrogenation is performed in the liquid phase in all reactors.

4. A process according to any of claims 1 to 3, characterized in that the flow is laminar in all reactors.

5. A process according to any of claims 1 to 3, characterized in that the flow is turbulent in all reactors.

6. A process according to any of claims 1 to 5, characterized in that the hydrogenation is carried out at temperatures of 40 to 150°C.

7. A process according to claim 6, characterized in that the hydrogenation is carried out at temperatures of 80 to 130°C.

8. A process according to any of claims 1 to 7, characterized in that the hydrogenation is carried out in the individual reactors at different temperature levels.

9. A process according to any of claims 1 to 8, characterized in that the hydrogenation is carried out at pressures of 1 to 100 bar absolute.

10. A process according to any of claim 9, characterized in that the hydrogenation is carried out at pressures of 5 to 20 bar absolute.

11. A process according to any of claims 1 to 10, characterized in that the hydrogenation is carried out in the individual reactors at different pressure levels.

12. A process according to any of claims 1 to 11, characterized in that the hydrogenation is carried out on a palladium catalyst.

13. A process according to claim 12, characterized in that the hydrogenation is carried out on a palladium catalyst which is applied to a support of aluminium oxide.

## Revendications

1. Procédé de fabrication du 2-éthylhex-2-énal,
caractérisé en ce que
l'hydrogénation est effectuée dans au moins deux boucles connectées l'une derrière l'autre, dans lequel le 2-éthylhex-2-énal conjointement à une partie du produit d'hydrogénation du premier réacteur est conduit sur la tête du premier réacteur, dans lequel la partie résiduelle du produit d'hydrogénation du premier réacteur conjointement à une partie du produit d'hydrogénation du 2ème réacteur est conduite sur la tête du 2ème réacteur et pour autant que plus de deux boucles soient prévues, la partie résiduelle du produit d'hydrogénation du 2ème réacteur conjointement à une partie du produit d'hydrogénation du réacteur suivant est conduite sur la tête du réacteur suivant etc ... et le 2-éthylhexanal est produit à partir de la partie résiduelle du produit d'hydrogénation du dernier réacteur.

2. Procédé selon la revendication 1,
caractérisé en ce que
l'hydrogénation est effectuée dans deux boucles connectées l'une derrière l'autre, dans laquelle le 2-éthylhex-2-énal conjointement à une partie du produit d'hydrogénation du premier réacteur est conduit sur la tête du premier réacteur, dans lequel la partie résiduelle du produit d'hydrogénation du premier réacteur conjointement à une partie du produit d'hydrogénation du deuxième réacteur est conduit sur la tête du deuxième réacteur, et le 2-éthylhexanal est produit à partir de la partie résiduelle du produit d'hydrogénation du deuxième réacteur.

3. Procédé selon les revendications 1 et 2,
caractérisé en ce qu'
on procède à une hydrogénation dans tous les réacteurs en phase liquide.

4. Procédé selon les revendications 1 à 3,
caractérisé en ce que
le courant dans tous les réacteurs est laminaire.

5. Procédé selon les revendications 1 à 3,
caractérisé en ce que
le courant dans tous les réacteurs est turbulent.

6. Procédé selon les revendications 1 à 5,
caractérisé en ce que
l'hydrogénation est effectuée à des températures allant de 40 à 150°C.

7. Procédé selon la revendication 6,
caractérisé en ce que
l'hydrogénation est effectuée à des températures allant de 80 à 130°C.

8. Procédé selon les revendications 1 à 7,
caractérisé en ce que
l'hydrogénation est effectuée dans des réacteurs individuels, à des niveaux de température différents.

9. Procédé selon les revendications 1 à 8,
caractérisé en ce que
l'hydrogénation est effectuée à des pressions allant de 1 à 100 bars abs.

10. Procédé selon la revendication 9,
caractérisé en ce que
l'hydrogénation est effectuée à des pressions allant de 5 à 20 bars abs.

11. Procédé selon les revendications 1 à 10,
caractérisé en ce que
l'hydrogénation est effectuée dans les réacteurs individuels à des niveaux de pression différents;

12. Procédé selon les revendications 1 à 11,
caractérisé en ce que
l'hydrogénation est effectuée sur un catalyseur au palladium.

13. Procédé selon la revendication 12,
caractérisé en ce que
l'hydrogénation est effectuée sur un catalyseur au palladium qui est appliqué sur un support en oxyde d'aluminium.
